# EUROPEAN PATENT SPECIFICATION

(11) **EP 1 214 078 B1**
(45) Date of publication and mention of the grant of the patent: **24.11.2004**
(21) Application number: 00967673.5
(22) Date of filing: 13.09.2000
(51) Int. Cl.: A61K 31/565, A61K 31/66, A61K 9/08, A61P 35/00, A61K 47/18

(54) **FORMULATIONS FOR PARENTERAL USE OF ESTRAMUSTINE PHOSPHATE AND AMINO ACIDS**
PARENTERAL ANZUWENDENDE ARZNEIZUSAMMENSETZUNGEN ENTHALTEND ESTRAMUSTINPHOSPHAT UND AMINOSÄURE
FORMULATIONS POUR UNE UTILISATION PARENTERALE DE PHOSPHATE D'ESTRAMUSTINE ET D'ACIDES AMINES

(30) Priority: 16.09.1999 GB 9921960
(43) Date of publication of application: 19.06.2002
(73) Proprietor: Pharmacia Italia S.p.A., 20152 Milano (IT)
(72) Inventor: MUGGETTI, Lorena, I-20036 Meda (IT); COLOMBO, Paolo, I-20147 Milan (IT); MARTINI, Alessandro, I-20149 Milan (IT); BUZZI, Giovanni, I-20131 Milan (IT)
(74) Representative: Modiano, Micaela Nadia
(86) International application number: PCT/EP2000/008983
(87) International publication number: WO 2001/019372

(56) References cited:
- EP-A- 0 612 530
- WO-A-00/59475
- US-A- 5 785 976
- US-A- 5 807 572

## Description

The present invention relates to pharmaceutical formulations of estramustine phosphate for parenteral use and, more particularly, to formulations of estramustine phosphate for parenteral use further comprising basic amino acids.

Estramustine phosphate (The Merck Index, XII Ed., No. 3749, 1996) is an estradiol-17β-phosphate derivative widely known in the art as antitumor agent, currently used in the treatment of advanced adenocarcinoma of the prostate.
The drug is usually administered orally, preferably at a dose of 10-15 mg/kg/day. Intravenous administration, however, is also adopted in some particular cases.
For example, initial intravenous administration of estramustine phosphate, followed by oral administration, has been reported at dosages paralleling the oral administration for the drug, i.e. 300-600 mg daily given intravenously and usually repetitively over for several consecutive days (see, for a reference, British Journal of Urology, 1977, 49, 73-79; J. Urol.108:303-306, 1972; Eur. Clin. Pharmacol. 26(1), 113-119, 1984; Eur. Urol. 1990, 17, 216-218).

Estramustine phosphate as well as other well-known cytotoxic compounds used in antitumor therapy are known to cause, or potentially cause, vascular damages at the site of injection when parenterally, in particular intravenously, administered.
As an example, studies in patients treated with estramustine phosphate administered as a slow intravenous injection or as a bolus, at 300 mg/day, revealed thrombophlebitis and local irritations at the peripheral intravenous injection sites.
These drawbacks are considered major limitations for the intravenous administration of estramustine phosphate, thus requiring, in many patients, the establishment of central line administration or, in some cases, even discontinuation of the treatment.

With the aim of minimising the unwanted effects associated with the intravenous administration of cytotoxic agents, a few means are reported in the art.
Among them is the use of cyclodextrins, for instance hydroxypropyl-cyclodextrin, in the preparation of formulations for parenteral administration of cytotoxic known to cause ulcerative lesions. See, for a reference, US patent No. 5,804,568 in the name of Supergen Inc.
Also known in the art are formulations for the intravenous administration of estramustine phosphate containing human albumin, reported to be characterised by fewer local side-effects upon injection of the active (see, for a reference, H. Schutz et al.; Krankenhauspharmazie, II year, issue No. 3, 1988).

In this respect, we found formulations for parenteral use comprising estramustine phosphate together with a basic amino acid which, unexpectedly, resulted to achieve optimal protection from side-effects associated with estramustine administration.

It is therefore the object of the present invention a formulation for parenteral use comprising estramustine phosphate and a basic amino acid.

Once administered intravenously to patients, the formulations object of the present invention do not provoke ulcerative damages, nor thrombophlebitis, at the site of injection.

In the present description, with the term basic amino acid we preferably intend a basic α-amino acid; preferred basic amino acids are selected from the group consisting of arginine, histidine and lysine.
Even more preferably, the formulations object of the present invention comprise arginine.
With the term formulation comprising estramustine phosphate we intend any formulation of estramustine phosphate, as the active ingredient, either in the acid form or as a pharmaceutically acceptable salt for parenteral use such as, for instance, the salts of estramustine phosphate with basic amino acids themselves or with N-methyl glucamine, otherwise referred to as meglumine.
Preferably, the formulations of the invention comprise estramustine phosphate in the form of its arginine or meglumine salt.

From the foregoing, it is clear to the man skilled in the art that the formulations of the present invention may contain the basic amino acid, either in the form of its salt with estramustine phosphate as well as in admixture with an estramustine phosphate salt for parenteral use.

According to a preferred embodiment of the invention, it is herewith provided a formulation for parenteral use comprising estramustine phosphate, in the form of its arginine salt, optionally in admixture with additional amounts of a basic amino acid, e.g. arginine.

Another preferred embodiment of the invention is a formulation for parenteral use comprising estramustine phosphate, in the form of its meglumine salt, in admixture with arginine.

In another preferred embodiment, estramustine phosphate is provided in lyophilised form and the basic amino acid is provided in physiological solution. Formulations of this type may typically be provided as a kit.

The formulations object of the present invention may further contain additional excipients for parenteral use.
Among them are also those excipients known in the art to reduce the side effects associated with the parenteral administration of cytotoxic agents (herewith referred to as protective agents) such as, for instance, human albumin, cyclodextrins, sulfoalkyl ether cyclodextrins and derivatives thereof.
Preferably, the formulations of the invention additionally comprise human albumin.

Very interestingly, the formulations of the invention also containing a given amount of human albumin are unexpectedly less toxic than the corresponding estramustine phosphate formulations containing the same amount of human albumin, as the sole protective agent against ulcerative damages and thrombophlebitis at the site of injection.

It is therefore a further object of the invention a formulation for parenteral use comprising estramustine phosphate and a basic amino acid in admixture with human albumin.

All of the above formulations are preferably intended for intravenous use. As such, they can be administered to patients either as a slow injection, e.g. over about 30 minutes to about 3 hours, or as a bolus injection, also referred to as IV (intravenous) push.

In addition, the formulations of the invention provide a very advantageous method for delivering estramustine phosphate intravenously, even when high doses of the active are needed.

It is therefore a further object of the invention a formulation for parenteral use comprising estramustine phosphate and a basic amino acid, wherein the active within a single infusion dosage form exceeds 1300 mg.
According to another preferred embodiment of the invention, it is further provided a formulation for parenteral use comprising estramustine phosphate and a basic amino acid, wherein the active within a single infusion dosage form exceeds 950 mg/m².

The formulations of the invention allow the administration of the active either as a single agent or, alternatively, according to a combined chemotherapy regimen.
As an example, the above formulations can be administered in combination with one or more chemotherapeutic agents such as, for instance, taxane derivatives, e.g. paclitaxel and docetaxel; camptothecin and derivatives thereof, e.g. CPT-11, 9-amino-camptothecin; anthracycline derivatives, e.g. doxorubicin, epirubicin, idarubicin, daunorubicin, alkycycline (4-demethoxy-3'-deamino-3'-aziridinyl-4'-methylsulfonyl-daunorubicin; internal code PNU 159548); etoposide; navelbine; vinblastine; platinum derivatives, e.g. carboplatin and cisplatin; angiogenesis inhibitors, e.g. Sugen SU-5416 and Sugen SU-6668; and the like, optionally within liposomal formulations thereof.

Therefore, it is a further object of the present invention a product containing a formulation for parenteral use comprising estramustine phosphate and a basic amino acid, preferably arginine, and one or more chemotherapeutic agents, as a combined preparation for simultaneous, separate or sequential use in anticancer therapy.

### Toxicology

To study the local irritant effects of estramustine phosphate (hereinafter referred to as EMP) after repeated intravenous administrations to rats, the active was prepared, according to the present invention, either in the form of a salt with a basic amino acid and/or in admixture with a basic amino acid. Subsequently, the said preparations were dissolved in different vehicles such as water solution for injection and water solution for injection further containing human albumin (hereinafter referred to as HA). In addition, formulations of estramustine phosphate in the form of meglumine (hereinafter referred to as MEG) salts, optionally in admixture with HA only, were used for comparison.
In particular, the following water for injection solutions (Table I) wherein either the active EMP as well as the basic amino acid or HA are expressed in terms of molar or weight ratios, were prepared and tested:

**Table I**

| **Solution** | **Molar ratio (mol/mol)** | **Weight ratio (w/w)** |
|---|---|---|
| **a)**negative control water for inject. | - | - |
| **b)**positive control EMP-MEG salt | EMP:MEG=1:1 | - |
| **c)**comparison EMP-MEG salt + HA | EMP:MEG=1:1 | EMP:HA=1:0.21 |
| **d)** EMP-Arg salt | EMP:Arg=1:1 | |
| **e)** EMP-Arg salt + Arg | EMP:Arg=1:2 | |
| **f)** EMP-Arg salt + HA | EMP:Arg=1:1 | EMP:HA=1:0.21 |
| **g)** EMP-MEG salt + Arg | EMP:MEG:Arg=1:1:2 | |

Male Sprague-Dawley rats were used because of their acceptance as a predictor of toxic change in man. The rats were 6 weeks old at the start of the study.

The above formulations were administered to groups of rats as a repeated intravenous injection during 3 days. Rats were then sacrificed: a half of the rats at the fourth day and a half at the fifth day.
The dose level of estramustine phosphate, in all the different tested solutions, was of 150 mg/kg/day.
Clinical observations were recorded daily. Thrombophlebitic side effects resulted in a dark bluish/blackish coloration of the tail during the treatment period.
A score system based on tail coloration and its extension was used to evaluate the different tested formulations.
The score system considered the formulation of estramustine phosphate salt with meglumine, solution **(b)**, as the positive control (i.e. marked toxicity). Water for injection **(a)** was administered to the control group as negative control (i.e. no toxicity signs).
Histological evaluation was carried out on the tail of the rats treated with the composition of the invention.
Estramustine phosphate in a water solution **(b)** induced, at the used dose, local irritant effects at the injection site after the first administration and marked toxicity signs at the end of the experiment.
Likewise, toxicity signs at the injection site were also observed for the comparison **(c)** EMP solution containing human albumin, as the sole protective excipient against ulcerative damages at the injection site.
On the contrary, a markedly reduced toxicity or even no toxicity was observed with the formulations of the invention containing arginine **(d), (e)** and **(g),** optionally in admixture with human albumin **(f).**
The histological evaluation of the tail of the rats treated with these formulations confirmed the above findings.
In addition, the protective effect exerted by the basic amino acid, according to the invention, can clearly be evidenced by considering the formulation **(f)** in comparison to the formulations **(c)** for which toxicity signs were observed. Very interestingly, in fact, both formulations contained the same amount of human albumin with respect to the active.

It was thus concluded that estramustine phosphate in a water solution containing a basic amino acid, either in the form of a salt with estramustine phosphate or in admixture with the same, induced markedly less local irritant effects when compared with a water solution of estramustine phosphate itself.
Even more surprisingly, the formulations of the invention produced less local irritant effects also in comparison to analogous solutions of estramustine phosphate containing known protective excipients, for instance human albumin.

One particularly preferred schedule for administering the formulation of estramustine phosphate according to the invention is a single infusion given once weekly to a maximal dose of 4000 mg or 3500 mg/m².
Another preferred schedule is the administration of a single drug infusion once every two to four weeks.
One schedule may be preferred over another in consideration of schedules with other optional concomitant therapy. These schedules may repeat in serial or as repetitive fashion.

The formulations of the present invention are useful in antitumor therapy, particularly in the treatment of prostate cancer, breast cancer, melanoma, lung cancer, pancreatic cancer, colorectal cancer, ovarian cancer and cancers of the brain.

In addition to the above, we also noticed that arginine and the pharmaceutically acceptable salts thereof for parenteral use, exerted their protective effects against the occurrence of thrombophlebitis also when used in combination with several other antineoplastic agents known to cause, or potentially cause, ulcerative damages at the site of injection, when administered intravenously.

Besides estramustine phosphate, other antineoplastic agents are known to cause, at least potentially, ulcerative damages and thrombophlebitis at the site of injection.
Preferably, these antineoplastic agents according to the invention are selected from the group consisting of anthracycline derivatives such as doxorubicin, epirubicin, idarubicin, daunorubicin and alkycycline (4-demethoxy-3'-deamino-3'-aziridinyl-4'-methylsulfonyl-daunorubicin;
internal code PNU 159548); and angiogenesis inhibitors such as Sugen SU-5416 and Sugen SU-6668.

From the foregoing, it is a further object of the invention the use of arginine, or pharmaceutically acceptable salts thereof, in the preparation of a medicament for the treatment and prevention of side-effects associated with the intravenous administration of antineoplastic agents.

As set forth above, these side-effects comprise ulcerative lesions and thrombophlebitis at the site of injection.

In the present description, unless otherwise specified, with the term pharmaceutically acceptable salt of arginine, we intend any salt for parenteral use including, preferably, the arginine addition salt with hydrochloric, glutamic or aspartic acid.

The formulations object of the present invention are prepared according to conventional techniques adopted in the preparation of pharmaceutical forms for parenteral use.

Typically, a proper amount of estramustine phosphate is dispersed in water and then dissolved by adding at least an equimolar amount of a basic amino acid, for instance arginine.
A further amount of the given amino acid, e.g. arginine, can be present in order to reach an estramustine phosphate:arginine molar ratio higher than 1:1, respectively.
Alternatively, a proper amount of estramustine phosphate in the form of a pharmaceutically acceptable salt for parenteral use, e.g. estramustine phosphate meglumine salt, either as a dry powder or into a lyophilised form, is dissolved in a pharmaceutically acceptable solution for parenteral use, for instance sterile water or aqueous dextrose solution, e.g. 5% dextrose in water for intravenous administration, and then admixed with a proper amount of a basic amino acid, for instance arginine.
The above admixture is then stirred, sterilised, and subsequently lyophilised according to conventional techniques.
The freeze-dried formulation is prepared and stored in vials for injection; the addition of a proper amount of sterile water or a physiological solution for parenteral use enables the preparation of the final formulation to be injected.

The above method is also suitable for preparing high dosages estramustine phosphate formulations whilst maintaining the desired weight or molar ratio between the components.
The unit strength of the formulation to be injected depended on the concentration of the active in the solution itself and, of course, on the filling volume of the vials used to prepare the final formulation.

Additionally, besides the aforementioned human albumin, cyclodextrins, sulfoalkyl ether cyclodextrins and derivatives thereof, the formulations of the invention may optionally contain additional pharmaceutically acceptable excipients for parenteral administration such as, for instance, bulking agents, e.g. lactose or mannitol, pH buffering agents, anti-oxidant agents, preservative agents, tonicity adjusters and the like.

From the foregoing, it is clear to the man skilled in the art that the above methods for preparing the estramustine formulations apply as well to arginine-containing formulations comprising antineoplastic agents other than estramustine phosphate, and known to cause, or potentially cause, thrombophlebitic side-effects upon intravenous administration.

The following examples are herewith intended to better illustrate the present invention.

### Example 1

### Preparation of estramustine phosphate N-methyl glucamine salt in admixture with human albumin (estramustine phosphate:meglumine=1:1 molar ratio, estramustine phosphate:albumin=1:0.21 weight ratio)

300 mg of estramustine phosphate were weighed in a beaker and dispersed by means of magnetic stirring in 5 ml of water. 120.8 mg of N-methyl-glucamine were then added under stirring to the watery dispersion of the active and, after a few minutes, a clear solution was obtained. 0.250 ml of a commercially available solution of human albumin at 25% concentration were added whilst maintaining the solution under stirring.
The obtained solution was then brought to the final volume of 10 ml with water so as to reach a final concentration of 30 mg/ml of estramustine phosphate and 6.25 mg/ml of human albumin (1:0.21 weight ratio respectively).

A solution prepared as previously described, properly sterilised by filtration, was tested for its local vein tolerability in rats.

### Example 2

The formulation described in Example 1 was also prepared by dissolving the commercially available Estracyt® freeze-dried formulation containing 300 mg/vial of the active. The reconstitution of the formulation was made by using 10 ml of a 6.25 mg/ml human albumin solution so as to obtain a final concentration of 30 mg/ml of estramustine phosphate and 6.25 mg/ml of human albumin (1:0.21 weight ratio respectively).
The albumin solution could be prepared either by dissolving in water a proper amount of human albumin as a dry powder or by properly diluting a commercially available human albumin solution.

### Example 3

### Preparation of estramustine phosphate arginine salt (estramustine phosphate:arginine=1:1 molar ratio)

300 mg of estramustine phosphate were weighed in a beaker and dispersed by means of magnetic stirring in 5 ml of water. 101 mg of arginine base were then added to the watery dispersion of the active whilst maintaining under stirring until a clear solution was obtained.
The prepared solution was then brought to the final volume of 10 ml with water so as to reach a final concentration of 30 mg/ml of estramustine phosphate and 10.1 mg/ml of arginine (1:1 molar ratio respectively).
A solution prepared as previously described, properly sterilised by filtration, was tested for its local vein tolerability in rats.

### Example 4

### Preparation of estramustine phosphate arginine salt in admixture with arginine (estramustine phosphate:arginine=1:2, molar ratio)

300 mg of estramustine phosphate were weighed in a beaker and dispersed by means of magnetic stirring in 5 ml of water. 202 mg of arginine base were then added to the watery dispersion of the active whilst maintaining under stirring until a clear solution was obtained. The basic pH of the obtained solution was brought to the physiological value of about 7.5 by slow addition of diluted hydrochloric acid.
The solution was then brought to the final volume of 10 ml with water so as to reach a final concentration of 30 mg/ml of estramustine phosphate and 20.2 mg/ml of arginine (1:2 molar ratio respectively).
A solution prepared as previously described, properly sterilised by filtration, was tested for its local vein tolerability in rats.

### Example 5

### Preparation of estramustine phosphate arginine salt in admixture with human albumin (estramustine phosphate:arginine=1:1 molar ratio, estramustine phosphate:albumin=1:0.21 weight ratio)

300 mg of estramustine phosphate were weighed in a beaker and dispersed by means of magnetic stirring in 5 ml of water. 101 mg of arginine base were then added to the watery dispersion of the active whilst maintaining under stirring until a clear solution was obtained. 0.250 ml of a commercially available solution of human albumin at 25% concentration were added, maintaining the solution under stirring.
The solution was then brought to the final volume of 10 ml with water so as to reach a final concentration of 30 mg/ml of estramustine phosphate, 10.1 mg/ml of arginine and 6.25 mg/ml of human albumin. The molar and weight ratios between the components of the solution were as follows: estramustine phosphate:arginine 1:1 molar ratio, estramustine phosphate:human albumin 1:0.21 weight ratio.
A solution prepared as previously described, properly sterilised by filtration, was tested for its local vein tolerability in rats.

### Example 6

### Preparation of estramustine phosphate N-methyl glucamine salt in admixture with arginine (estramustine phosphate:meglumine:arginine=1:1:2 molar ratio)

300 mg of estramustine phosphate were weighed in a beaker and dispersed by means of magnetic stirring in 5 ml of water. 120.8 mg of N-methyl-glucamine were then added under stirring to the watery dispersion of the active and, after a few minutes, a clear solution was obtained. A total amount of 202 mg of arginine was then added to the above described solution by using a suitable mixture of arginine base and hydrochloride, in order to maintain the pH around the physiological value of 7.5.
The solution was then brought to the final volume of 10 ml with water so as to reach a final concentration of 30 mg/ml of estramustine phosphate and 20.2 mg/ml of arginine (1:2 molar ratio respectively).
A solution prepared as previously described, properly sterilised by filtration, was tested for its local vein tolerability in rats.

### Example 7

The formulation described in Example 6 was also prepared by solubilization of the commercially available Estracyt® freeze-dried formulation containing 300 mg/vial of the active. The reconstitution of the formulation was made by using 10 ml of a 20.2 mg/ml arginine solution so as to reach a final concentration of 30 mg/ml of estramustine phosphate and 20.2 mg/ml of arginine (1:2 molar ratio respectively).

The arginine solution used to dissolve the freeze-dried formulation contained a proper mixture of arginine base and hydrochloride, in order to maintain the pH around the physiological value of 7.5.

### Example 8

### Preparation of a formulation comprising doxorubicin and arginine in a 1:1 molar ratio.

Doxorubicin hydrochloride (40 mg) and arginine (12 mg) were weighed in a flask and the admixture was dissolved in a physiological solution (15 ml) of NaCl 0.9% w/v, under stirring. A solution of hydrochloric acid was then added up to pH = 3. The solution thus prepared was then diluted with the above physiological solution up to a final volume of 20 ml so as to reach a final concentration of 2 mg/ml of doxorubicin and 0.6 mg/ml of arginine (molar ratio 1:1, respectively).

### Example 9

### Preparation of a formulation comprising doxorubicin and arginine in a 1:2 molar ratio

By working as above described in example 8 and by using an amount of arginine as twice, that is 24 mg of arginine per 40 mg of doxorubicin hydrochloride, a solution comprising doxorubicin and arginine in a molar ratio 1:2, respectively, was thus prepared.

### Example 10

### Preparation of a formulation comprising Sugen SU-5416 and arginine in a 1:1 molar ratio

10 ml of an aqueous solution of NaCl (0.9% w/v) were diluted with 10 ml of water in a flask so as to obtain a solution of NaCl at 0.45% w/v.
Arginine hydrochloride (39.79 mg) was then added to the resultant solution and dissolved by shaking.
The solution thus prepared was used to dilute a solution of Sugen SU-5416 having the following composition:

| Component | Amount % (w/v) in the formulation |
|---|---|
| Sugen SU 5416 | 0.45 % |
| PEG 400 | 45 % |
| Benzyl alcohol | 2 % |
| Cremophor EL | 31.5 % |
| Anhydrous Ethanol | Up to 100 |

## Claims

1. A pharmaceutical formulation which comprises a parenterally acceptable carrier or diluent and estramustine phosphate and a basic amino acid.

2. A formulation according to claim 1 wherein the basic amino acid is arginine.

3. A formulation according to claim 1 or 2 wherein estramustine phosphate is in the form of a pharmaceutically acceptable salt for parenteral use.

4. A formulation according to claim 3 wherein the estramustine phosphate is in the form of a salt with arginine, histidine, lysine or N-methyl glucamine.

5. A formulation according to claim 4 wherein the estramustine phosphate is in the form of a salt with arginine or N-methyl glucamine.

6. A formulation according to anyone of the preceding claims comprising estramustine phosphate and arginine in a molar ratio lower than 1:1.

7. A formulation according to claim 6 comprising estramustine phosphate and arginine in a molar ratio of 1:2.

8. A formulation according to claim 1 which further comprises human albumin, a cyclodextrin or a sulfoalkyl ether cyclodextrin.

9. A formulation according to claim 1 which further comprises human albumin.

10. A formulation according to any one of the preceding claims wherein a single infusion dosage form at least comprises 1300 mg of estramustine phosphate:

11. A formulation according to any one of the preceding claims wherein a single infusion dosage form at least comprises 950 mg/m² of estramustine phosphate.

12. A formulation according to claim 10 or 11 wherein the basic amino acid is arginine.

13. A formulation according to any one of the preceding claims for intravenous use.

14. A formulation according to any one of the preceding claims for use in the treatment of cancer.

15. A formulation as claimed in claim 14 wherein the cancer is prostate cancer, breast cancer, melanoma, lung cancer, pancreatic cancer, colorectal cancer, ovarian cancer or cancer of the brain.

16. A formulation according to claim 1 wherein the parenterally acceptable carrier is a physiological solution for parenteral use which contains the basic amino acid, and the estramustine phosphate is in lyophilised form.

17. A product which comprises:
(i) a pharmaceutical formulation which comprises a parenterally acceptable carrier or diluent and estramustine phosphate and a basic amino acid, and
(ii) one or more chemotherapeutic agents selected from the group consisting of taxane derivatives such as paclitaxel and docetaxel; camptothecin and derivatives thereof such as CPT-11, 9-amino-camptothecin; anthracycline derivatives such as doxorubicin, epirubicin, idarubicin, daunorubicin, alkycycline (4-demethoxy-3'-deamino-3'-aziridinyl-4'-methylsulfonyl-daunorubicin; internal code PNU 159548); etoposide; navelbine; vinblastine; platinum derivatives such as carboplatin and cisplatin; angiogenesis inhibitors such as Sugen SU-5416 and Sugen SU-6668; optionally within liposomal formulations thereof,
as a combined preparation for simultaneous, separate or sequential use in anticancer therapy.

18. A product according to claim 17 wherein the basic amino acid is arginine.

19. A product according to claim 17 for intravenous use.

20. A product according to claim 17 for use in the treatment of prostate cancer, breast cancer, melanoma, lung cancer, pancreatic cancer, colorectal cancer, ovarian cancer or cancer of the brain.

21. A formulation as defined in claim 13 for use in suppressing or reducing the side-effects associated with the intravenous administration of estramustine phosphate and pharmaceutically acceptable salts thereof.

22. A formulation according to claim 21 wherein the side effects comprise ulcerative lesions and thrombophlebitis at the site of injection.

23. A product which comprises estramustine phosphate in lyophilised form and a physiological solution for parenteral use containing a basic amino acid as a combined preparation for simultaneous, separate or sequential use in intravenous cancer treatment.

24. Use, in the manufacture of a medicament for parenteral administration, of estramustine phosphate and a basic amino acid.

25. Use according to claim 24 wherein the medicament is for intravenous administration.

26. Use of arginine, or of pharmaceutically acceptable salts thereof, in the preparation of a medicament for the treatment and prevention of side-effects associated with the intravenous administration of antineoplastic agents.

27. Use according to claim 26 wherein the side-effects comprise ulcerative lesions and thrombophlebitis at the site of injection.

28. Use according to claim 26 wherein the antineoplastic agent is selected from the group consisting of estramustine phosphate; anthracycline derivatives such as doxorubicin, epirubicin, idarubicin, daunorubicin and alkycycline (4-demethoxy-3'-deamino-3'-aziridinyl-4'-methylsulfonyl-daunorubicin; internal code PNU 159548); and angiogenesis inhibitors such as Sugen SU-5416 and Sugen SU-6668.

## Patentansprüche

1. Eine pharmazeutische Formulierung, welche einen parenteral verträglichen Träger oder ein Verdünnungsmittel und Extramustinphosphat und eine basische Aminosäure umfaßt.

2. Eine Formulierung gemäß Anspruch 1, worin die basische Aminosäure Arginin ist.

3. Eine Formulierung gemäß Anspruch 1 oder 2, worin Estramustinphosphat in der Form eines pharmazeutisch verträglichen Salzes für die parenterale Verwendung vorliegt.

4. Eine Formulierung gemäß Anspruch 3, worin das Estramustinphosphat in der Form eines Salzes mit Arginin, Histidin, Lysin oder N-Methylglucamin vorliegt.

5. Eine Formulierung gemäß Anspruch 4, worin das Estramustinphosphat in der Form eines Salzes mit Arginin oder N-Methylglucamin vorliegt.

6. Eine Formulierung gemäß irgendeinem der vorhergehenden Ansprüche, die Estramustinphosphat und Arginin in einem Molverhältnis von weniger als 1:1 umfaßt.

7. Eine Formulierung gemäß Anspruch 6, die Extramustinphosphat und Arginin in einem Molverhältnis von 1:2 umfaßt.

8. Eine Formulierung gemäß Anspruch 1, welche weiterhin Humanalbumin, ein Cyclodextrin oder ein Sulfoalkylether-Cyclodextrin umfaßt.

9. Eine Formulierung gemäß Anspruch 1, welche weiterhin Humanalbumin umfaßt.

10. Eine Formulierung gemäß irgeneinem der vorhergehenden Ansprüche, worin eine einzelne Infusionsdosierform mindestens 1300 mg Estramustinphosphat umfaßt.

11. Eine Formulierung gemäß irgendeinem der vorhergehenden Ansprüche, worin eine einzelne Infusionsdosierform mindestens 950 mg/m² Estramustinphosphat umfaßt.

12. Eine Formulierung gemäß Anspruch 10 oder 11, worin die basische Aminosäure Arginin ist.

13. Eine Formulierung gemäß irgendeinem der vorhergehenden Ansprüche für die intravenöse Verwendung.

14. Eine Formulierung gemäß irgendeinem der vorhergehenden Ansprüche zur Verwendung bei der Behandlung von Krebs.

15. Eine Formulierung wie in Anspruch 14 beansprucht, worin der Krebs Prostatakrebs, Brustkrebs, Melanom, Lungenkrebs, Bauchspeicheldrüsenkrebs, Kolorektalkrebs, Eierstockkrebs oder ein Gehirntumor ist.

16. Eine Formulierung gemäß Anspruch 1, worin der parenteral verträgliche Träger eine physiologische Lösung für die parenterale Verwendung ist, welche die basische Aminosäure enthält, und das Estramustinphosphat ist in lyophilisierter Form.

17. Ein Produkt, welches folgendes umfaßt:
(i) eine pharmazeutische Formulierung, welche einen parenteral verträglichen Träger oder Verdünnungsmittel und Estramustinphosphat und eine basische Aminosäure umfaßt, und
(ii) einen oder mehrere chemotherapeutische Wirkstoffe, gewählt aus der Gruppe bestehend aus Taxanderivaten, wie zum Beispiel Paclitaxel und Docetaxel; Camptothecin und Derivate davon, wie zum Beispiel CPT-11, 9-Aminocamptothecin; Anthracyclinderivate, wie zum Beispiel Doxorubicin, Epirubicin, Idarubicin, Daunorubicin, Alkycyclin (4-Demetoxy-3'-desamino-3'-aziridinyl-4'-methylsulfonyl-daunorubicin; interner Code PNU 159548); Etoposid; Navelbin; Vinblastin; Platinderivate, wie zum Beispiel Carboplatin und Cisplatin; Angiogeneseinhibitoren, wie zum Beispiel Sugen SU-5416 und Sugen SU-6668; wahlweise innerhalb liposomaler Formulierungen davon,
als eine kombinierte Zubereitung für die gleichzeitige, separate oder aufeinanderfolgende Verwendung in der Antikrebstherapie.

18. Ein Produkt gemäß Anspruch 17, worin die basische Aminosäure Arginin ist.

19. Ein Produkt gemäß Anspruch 17, für die intravenöse Verwendung.

20. Ein Produkt gemäß Anspruch 17, für die Verwendung bei der Behandlung von Prostatakrebs, Brustkrebs, Melanom, Lungenkrebs, Bauchspeicheldrüsenkrebs, Kolorektalkrebs, Eierstockkrebs oder Gehirntumor.

21. Eine Formulierung wie in Anspruch 13 definiert, für die Verwendung in der Supprimierung oder Reduktion der Nebenwirkungen, die mit der intravenösen Verabreichung von Estramustinphosphat und pharmazeutisch verträglichen Salze davon verbunden sind.

22. Eine Formulierung gemäß Anspruch 21, worin die Nebenwirkungen ulzerative Laesionen und Thrombophlebitis an der Injektionsstelle umfassen.

23. Ein Produkt, welches Estramustinphosphat in lyophilisierter Form und eine physiologische Lösung für die parenterale Verwendung umfaßt, die eine basische Aminosäure enthält, als eine kombinierte Zubereitung für die gleichzeitige, separate oder aufeinanderfolgende Verwendung in intravenösen Krebsbehandlungen.

24. Verwendung von Estramustinphosphat und einer basischen Aminosäure in der Herstellung eines Medikaments für die parenterale Verabreichung.

25. Verwendung gemäß Anspruch 24, worin das Medikament für die intravenöse Verabreichung ist.

26. Verwendung von Arginin oder von pharmazeutisch verträglichen Salzen davon, in der Herstellung eines Medikaments für die Behandlung und Prävention von Nebenwirkungen, die mit der intravenösen Verabreichung von antineoplastischen Wirkstoffen verbunden sind.

27. Verwendung gemäß Anspruch 26, worin die Nebenwirkungen ulzerative Laesionen und Thrombophlebitis an der Injektionsstelle umfassen.

28. Verwendung gemäß Anspruch 26, worin der antineoplastische Wirkstoff gewählt ist aus der Gruppe bestehend aus Estramustinphosphat; Anthracyclinderivaten, wie zum Beispiel Doxorubicin, Epirubicin, Idarubicin, Daunorubicin und Alkycyclin (4-Demethoxy-3'-desamino-3'-aziridinyl-4'-methylsulfonyldaunorubicin; interner Code PNU 159548); und Angiogeneseinhibitoren, wie zum Beispiel Sugen SU-5416 und Sugen SU-6668.

## Revendications

1. Formulation pharmaceutique qui comprend un support ou diluant acceptable du point de vue parentéral et du phosphate d'estramustine et un aminoacide basique.

2. Formulation suivant la revendication 1, dans laquelle l'aminoacide basique est l'arginine.

3. Formulation suivant la revendication 1 ou 2, dans laquelle le phosphate d'estramustine est sous forme d'un sel pharmaceutiquement acceptable à usage parentéral.

4. Formulation suivant la revendication 3, dans laquelle le phosphate d'estramustine est sous forme d'un sel avec l'arginine, l'histidine, la lysine ou la N-méthylglucamine.

5. Formulation suivant la revendication 4, dans laquelle le phosphate d'estramustine est sous forme d'un sel avec l'arginine ou la N-méthylglucamine.

6. Formulation suivant l'une quelconque des revendications précédentes, comprenant du phosphate d'estramustine et de l'arginine en un rapport molaire inférieur à 1:1.

7. Formulation suivant la revendication 6, comprenant du phosphate d'estramustine ét de l'arginine en un rapport molaire de 1:2.

8. Formulation suivant la revendication 1, qui comprend en outre de l'albumine humaine, une cyclodextrine ou un éther sulfoalkylique de cyclodextrine.

9. Formulation suivant la revendication 1, qui comprend en outre de l'albumine humaine.

10. Formulation suivant l'une quelconque des revendications précédentes, dans laquelle une forme posologique pour perfusion unique comprend au moins 1 300 mg de phosphate d'estramustine.

11. Formulation suivant l'une quelconque des revendications précédentes, dans laquelle une forme posologique pour perfusion unique comprend au moins 950 mg/m² de phosphate d'estramustine.

12. Formulation suivant la revendication 10 ou 11, dans laquelle l'aminoacide basique est l'arginine.

13. Formulation suivant l'une quelconque des revendications, à usage intraveineux.

14. Formulation suivant l'une quelconque des revendications précédentes, destinée à être utilisée dans le traitement du cancer.

15. Formulation suivant la revendication 14, dans laquelle le cancer est le cancer de la prostate, le cancer du sein, un mélanome, le cancer du poumon, le cancer du pancréas, le cancer colorectal, le cancer des ovaires ou le cancer du cerveau.

16. Formulation suivant la revendication 1, dans laquelle le support acceptable du point de vue parentéral est une solution physiologique à usage parentéral qui contient l'aminoacide, et le phosphate d'estramustine est sous forme lyophilisée.

17. Produit qui comprend :
(i) une formulation pharmaceutique qui comprend un support ou diluant acceptable du point de vue parentéral et du phosphate d'estramustine et un aminoacide basique, et
(ii) un ou plusieurs agents chimiothérapeutiques choisis dans le groupe consistant en des dérivés de taxane tels que le paclitaxel et le docétaxel ; la camptothécine et ses dérivés tels que le CPT-11, la 9-aminocamptothécine ; des dérivés d'anthracycline tels que la doxorubicine, l'épirubicine, l'idarubicine, la daunorubicine, l'alkycycline (4-déméthoxy-3'-désamino-3'-aziridinyl-4'-méthylsulfonyl-daunorubicine ; code interne PNU159548) ; l'étoposide ; la havelbine ; la vinblastine ; des dérivés de platine tels que le carboplatine et le cisplatine ; des inhibiteurs d'angiogénèse tels que le Sugen SU-5416 et le Sugen SU-6668 ; éventuellement dans des formulations liposomales de ces agents,
sous forme d'une préparation combinée pour une utilisation simultanée séparée ou séquentielle en thérapie anticancéreuse.

18. Produit suivant la revendication 17, dans lequel l'aminoacide basique est l'arginine.

19. Produit suivant la revendication 17, à usage intraveineux,

20. Produit suivant la revendication 17, destiné à être utilisé dans le traitement du cancer de la prostate, du cancer du sein, du mélanome, du cancer du poumon, du cancer du pancréas, du cancer colorectal, du cancer des ovaires ou du cancer du cerveau.

21. Formulation suivant la revendication 13, destinée à être utilisée dans la suppression ou la réduction des effets secondaires associés à l'administration intraveineuse de phosphate d'estramustine et de ses sels pharmaceutiquement acceptables.

22. Formulation suivant la revendication 21, dans laquelle les effets secondaires comprennent des lésions ulcératives et une thrombophlébite au site d'injection.

23. Produit qui comprend du phosphate d'estramustine sous forme lyophilisée et une solution physiologique à usage parentéral contenant un aminoacide basique, sous forme d'une préparation combinée pour l'utilisation simultanée séparée ou séquentielle dans les traitements intraveineux du cancer.

24. Utilisation, dans la production d'un médicament pour administration parentérale, de phosphate d'estramustine et d'un aminoacide basique.

25. Utilisation suivant la revendication 24, dans laquelle le médicament est destiné à l'administration intraveineuse.

26. Utilisation d'arginine, ou de ses sels pharmaceutiquement acceptables, dans la préparation d'un médicament destiné au traitement et à la prévention des effets secondaires associés à l'administration intraveineuse d'agents antinéoplasiques.

27. Utilisation suivant la revendication 26, dans laquelle les effets secondaires comprennent des lésions ulcératives et une thrombophlébite au site d'injection.

28. Utilisation suivant la revendication 26, dans laquelle l'agent antinéoplasique est choisi dans le groupe consistant en le phosphate d'estramustine ; des dérivés d'anthracycline tels que la doxorubicine, l'épirubicine, l'idarubicine, la daunorubicine et l'alkycycline (4-déméthoxy-3'-désamino-3'-aziridinyl-4'-méthylsulfonyl-daunorubicine ; code interne PNU 159548) ; et des inhibiteurs d'angiogénèse tels que le Sugen SU-5416 et le Sugen SU-6668.
